# EUROPÄISCHE PATENTANMELDUNG

(11) **EP 2 368 500 A1**
(43) Veröffentlichungstag der Anmeldung: **28.09.2011**
(21) Anmeldenummer: 11002343.9
(22) Anmeldetag: 22.03.2011
(51) Int. Cl.: A61B 17/02, A61B 1/32, A61B 19/00

(54) **Chirurgischer Retraktor und Verwendung im Rahmen einer Thorakoskopischen Operation**

(30) Priorität: 23.03.2010 DE 102010012586
(71) Anmelder: W.O.M. World of Medicine AG, 10587 Berlin (DE)
(72) Erfinder: Winterberg, Holger, 10247 Berlin (DE); Wilke, Marsha, 10781 Berlin (DE); Gelbert, Nils, 12555 Berlin (DE); Weifle, Udo, Dr., 64646 Heppenheim/Bergstrasse (DE)
(74) Vertreter: Seuss, Thomas

(57) **Zusammenfassung**

Die vorliegende Patentanmeldung betrifft einen neuen chirurgischen Retraktor und dessen Verwendung im Rahmen von thorakoskopischen Operationen.

## Beschreibung

Die vorliegende Patentanmeldung betrifft einen neuen chirurgischen Retraktor und dessen Verwendung im Rahmen von thorakoskopischen Operationen.

### Hintergrund und Stand der Technik

Mit zunehmender Häufigkeit operieren Herz- und Thoraxchirurgen endoskopisch. Aus Platzgründen wird bei derartigen Operationen im Thoraxinneren oft nur ein Lungenflügel beatmetet. Diese sogenannte Ein-Lungen-Ventilation birgt für den Patienten Risiken und verringert die minimal-Invasivität der Operationsmethode. Um ein Beispiel anzuführen: Es handelt es sich bei dem betroffenen Patientenklientel z.T. um Patienten mit schwer eingeschränkter Herzfunktion, so dass die thorakalen Druckverhältnisse unter Ein-Lumen-Ventilation die Hämodynamik deutlich verschlechtern und das Risiko für den Patienten erhöht. Um dieses zu minimieren wurde das erfindungsgemäße Instrument entwickelt. Dessen Hauptfunktion es ist während einer Operation im Thorax den beatmeten linken Lungenflügel zurückzuhalten. Dies kann wichtig sein beispielsweise bei der Herz-Schrittmachertechnologie, der kardialen Resynchronisations-Therapie, wobei eine epikardiale Schrittmachersonde direkt auf dem Herzen befestigt wird und durch die Erfindung eine sichere Applikation der Elektrode ermöglicht wird.

Aus dem Stand der Technik sind sogenannte endoskopische Fächerretraktoren bekannt, welche einen Fächer bestehend aus mehreren Lamellen aufweisen. Bei den Retraktoren unterscheidet man Fingerretraktoren (3 oder 5 Finger) (engl. FAN retractor) und Retraktoren die mit Hilfe innenliegender Seilzüge eine krümmbare Kette aufspannen (z.B. original von Fa. snowden pencer, siehe auch US 6,309,349).

Diese Fächerretraktoren dienen zu operativen Eingriffen des Bauchraumes, wobei sie z.B. dazu dienen die Leber wegzuhalten. Die bekannten Fächerretraktoren sind allerdings zu den oben beschriebenen Eingriffen in Herznähe nicht geeignet, da sie die Lunge verletzen könnten.

### Beschreibung der Erfindung

Es wurde gefunden, dass ein chirurgischer Retraktor mit einem Griffteil am lateralen und einer sich ein- und ausfahrbaren Fläche am distalen Ende hervorragend dafür geeignet ist, die eingangs geschilderten Probleme zu überwinden.

Der erfindungsgemäße Retraktor besteht aus einem länglichen Schaft (1) und einem Handgriff (2). Der Schaft hat eine ovale Form und beinhaltet zwei durchgängige Lumen. Ein Lumen dient der Aufnahme einer 5 mm Endoskopoptik, das andere Lumen beinhaltet eine ausfahrbare und aufspannbare Spateleinheit (3). An das proximale Ende des Schafts kann ein Insufflationsschlauch angeschlossen werden. Das Insufflationsgas kann über beide Lumen vom proximalen Ende des Schafts zum distalen Ende des Schafts strömen und gelangt so in den Operationsraum. Das proximale Schaftende wird mit dem distalen Handgriffende fest verbunden.

Der Handgriff beinhaltet Bedienelemente, mit denen die Spateleinheit ausgefahren, aufgespannt und zusammengeklappt werden kann. Weiterhin beinhaltet der Handgriff alle mechanischen Bauteile, die für die Umsetzung der Bedienfunktionen notwendig sind. Die Endkappe des Handgriffs (2.3) ist so gestaltet, dass ein eingestecktes Endoskop (I) einrastet und gegen translatorische und rotatorische Bewegungen fixiert ist. In Handgriff und Endkappe liegt ein Rohr (II), durch das das Endoskop problemlos in den Schaft eingeführt werden kann. Der kleine Spalt zwischen Endoskop und Rohr hat gleichzeitig die Wirkung einer Dichtung. Die Endkappe wird in verschiedenen Längen hergestellt, um die Verwendung von unterschiedlich langen Standardoptiken zu ermöglichen. Die Endkappe ist so gestaltet, dass sie einen Standardluerlockanschluss des Insufflationsschlauchs aufnehmen kann.

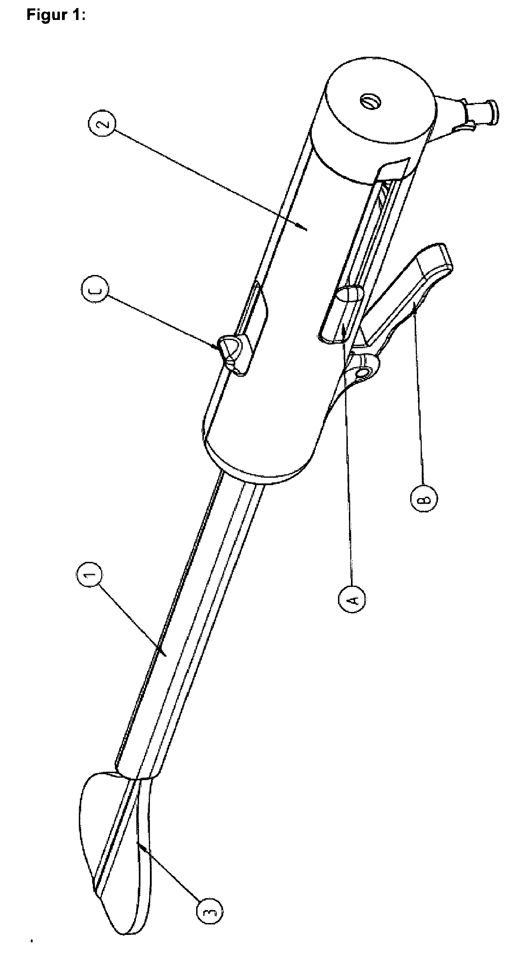

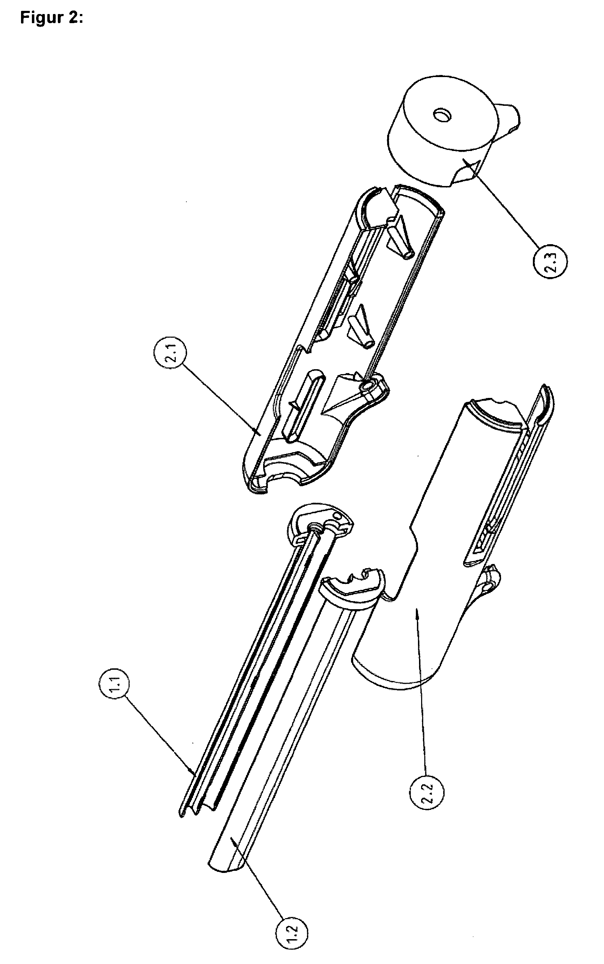

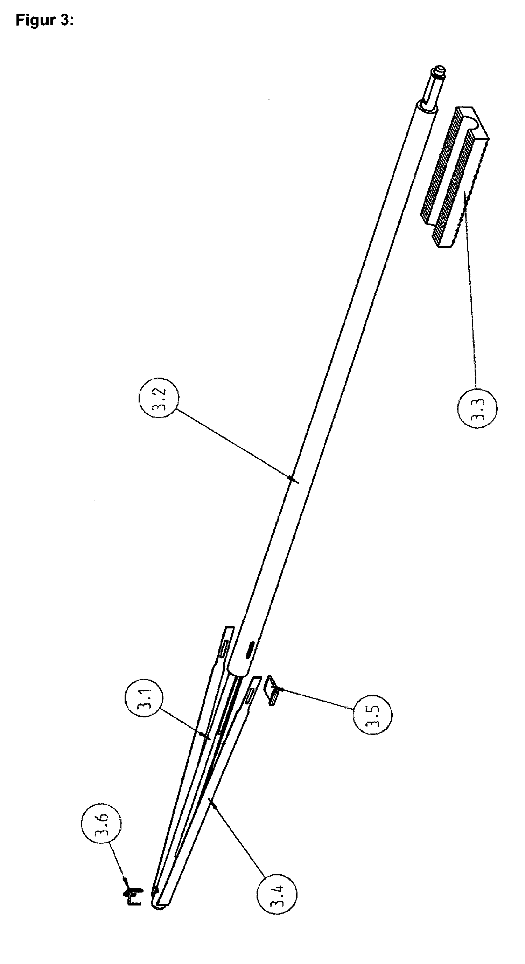

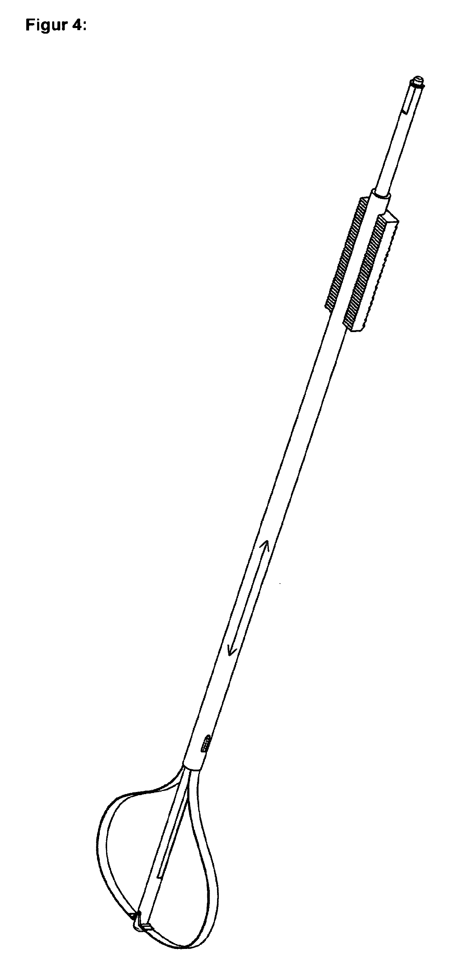

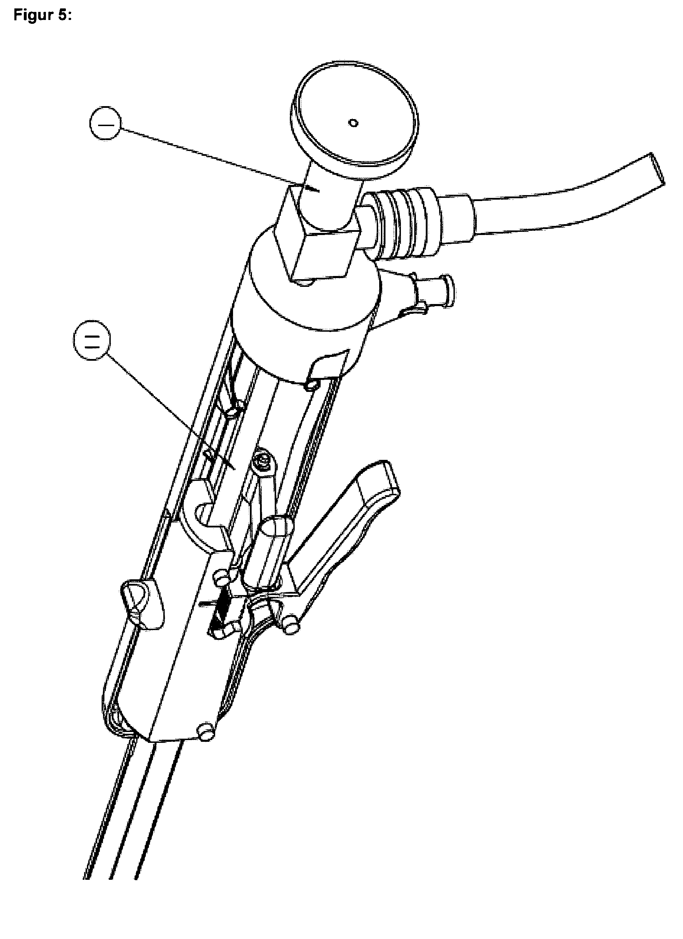

Die Spateleinheit besteht aus einer Schubstange (3.1) die in einem Rohr (3.2) steckt. Der Außendurchmesser der Schubstange ist kleiner als der Innendurchmesser des Rohres. Beide Teile können sich relativ zueinander bewegen. Die distalen Enden von Schubstange und Rohr sind über die beiden Spreizbleche (3.4) verbunden. Wird der Abstand der distalen Enden von Schubstange und Innenrohr vergrößert, legen sich die Spreizbleche dicht an die Schubstange an. Der Spatel ist geschlossen. Wird durch eine Relativbewegung der Abstand zwischen den distalen Enden von Schubstange und Innenrohr verkleinert, wölben sich die Spreizbleche nach außen. Der Spatel ist geöffnet (Abbildung 4). Ein Querriegel fixiert die Spreizbleche am Innenrohr und verhindert eine Verdrehung der Teile zueinander. Über die Spreizbleche ist ein hoch elastischer Stoff gespannt, der für eine geschlossene Oberfläche des Spatels sorgt, welcher im Einsatz die Retraktion der Lunge bewirkt.

Das Griffteil dient zur Manipulation der Fläche und ermöglicht die Aufnahme verschiedener handelsüblicher 5 mm Optiken diverser Firmen.

Der eigentliche Retraktor, wird durch die aufgespannte Fläche des Spatels (3) gebildet. Je nach Einsatz des Gerätes (s.u.) kann die Fläche unterschiedliche Größe haben, z.B. einen maximalen Durchmesser von 5 -20 cm haben.

### Verwendungen

Der erfindungsgemäße Retraktor wird bevorzugt im Bereich der Thoraxchirurgie eingesetzt. Das Instrument wird dazu im geschlossenen Zustand durch eine Inzision im Brustkorb in den Thorax eingeführt. Mit Hilfe eines Bedienelements A wird die geschlossene Spateleinheit kontrolliert ausgefahren. Mit Hilfe eines Bedienelements B wird die Spateleinheit kontrolliert aufgespannt, wobei die Spreizbleche arretiert werden, so dass die Spateleinheit im jeweils aufgespannten Stadium verharrt. Der ausgefahrene und aufgespannte Spatel hält Gewebe und Organe so aus dem Operationsfeld, dass eine freie Sicht auf die gewünschte Region gewährleistet wird. Hierzu muss der Spatel einem Widerstand von ca. 25mmHg standhalten können. Mit Hilfe eines Bedienelements C kann die Spateleinheit durch Aufhebung der Arretierung so weit zusammen geklappt werden, dass das Instrument ohne Komplikationen aus dem Thorax herausgezogen werden kann. Im Regelfall ist die Federspannnung, unter der die Spreizbleche stehen, ausreichend um das vollständige Zusammenklappen der Spateleinheit nach Betätigung des Bedienknopfes C zu bewirken. Alle Schritte werden mit Hilfe einer in das Gerät eingeführten Endoskopoptik unter Sichtkontrolle durchgeführt.

Andere Einsatzgebiete des Retraktors sind möglich, beispielsweise im Rahmen von endoskopischen Operationen im Abdomen, bei denen beispielsweise eine Retraktion der Leber, des Darms oder anderer Organe erforderlich ist.

Gegenüber den aus dem Stand der Technik bekannten Fächerretraktoren, weist der erfindungsgemäße Retraktor eine Reihe von Vorteilen auf. Dazu gehört die geringere Verletzungsgefahr, welche den Einsatz in Herznähe erlauben, aber auch auf anderen Verwendungsgebieten Vorteile bietet. Ein weiterer Vorteil ist die Möglichkeit der einfachen Bedienung. Zunächst kann die Bedienung einhändig erfolgen, so dass der verantwortliche Chirurg die zweite Hand für andere Zwecke nutzen kann. Insbesondere bemerkenswert ist die einfache Möglichkeit den aufgespannten Retraktor durch Betätigung des Bedienelements C (Release-knopf) schnell einzuklappen (durch Aufhebung der oben genannten Arretierung, worauf der Spatel durch die Federwirkung der Spreizbleche wieder in den Ursprungszustand zurückkehrt) um die Bergung des Instrumentes zu ermöglichen. Dies ist insbesondere bei intraoperativen Notfällen von besonderer Bedeutung.

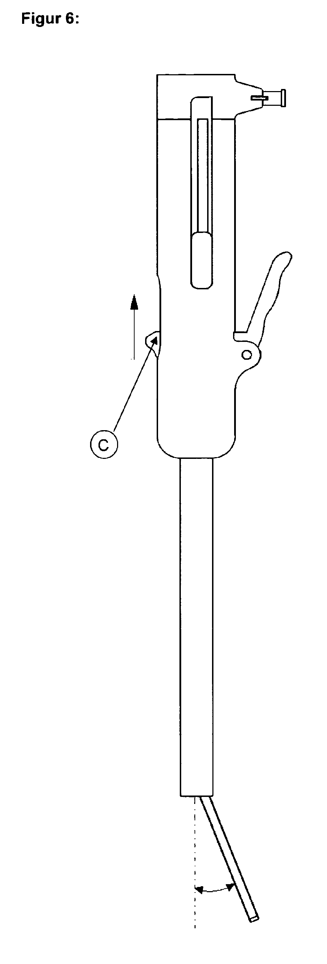

In einer weiteren Ausgestaltung der Erfindung kann der Spatel im aufgespannten Zustand um 30 - 60° aus der Instrumentenachse abgewinkelt werden. Die Abwinkelung erfolgt über die innen liegende Mechanik. Auch diese optionale Abwinkelungsvorrichtung ist einhändig bedienbar und insbesondere durch Betätigung des (eines) Releaseknopfes C in einfacher Weise rückstellbar für den Fall einer intraoperativen Notsituation. Eine Darstellung einer solchen möglichen Ausführungsform ist in Figur 6 dargestellt. Durch Betätigung des Bedienelements C (Release-knopf) in Pfeilrichtung wird der abgeklappte Spatel durch Federspannung wieder in die Instrumentenachse (gestrichelte Linie) zurückgeklappt, so dass das Instrument im Notfall schnell geborgen werden kann.

Alternativ kann der Spatel auch durch Auffächerung von Lamellen aufgespannt werden. Diese Lamellen können ihrerseits mit Stoff bespannt sein. Bei voller Aufspannung des Fächers kann die Länge der längsten Lamelle 100 mm betragen. Vorzugsweise wird die Länge maximal 80 mm sein. Die maximale Breite des Fächers beträgt 150-180 mm. Der maximale Fächerwinkel bezogen auf die Längsachse des Retraktorschaftes beträgt 45° zu beiden Seiten.

Der Gesamtdurchmesser des Retraktors im Bereich des Schaftes beträgt 8-12 mm, vorzugsweise 11 mm. Der Schaft kann eine Länge von 300 - 400 mm aufweisen.

Der erfindungsgemäße Retraktor enthält im Retraktorschaft einen Arbeitskanal zur Aufnahme einer handelsüblichen Optik. Hierbei kann es sich beispielsweise um eine handelsübliche Stablinsenoptik mit 5 mm Durchmesser und 30° Aufnahmewinkel von 350 mm Länge handeln. Eine derartige Stablinsenoptik ermöglicht die visuelle Beurteilung des Operationsfeldes. Alternativ kann auch ein flexibles Endoskop oder ein starres oder flexibles Endoskop mit Videobildsensor (bspw. CCD oder C-MOS) am distalen Ende Verwendung finden.

In einer weiteren Ausgestaltung des erfindungsgemäßen Retraktors enthält der Schaft einen weiteren Arbeitskanal, der zur Insufflation verwendet werden kann. Der optionale Insufflationskanal dient beispielsweise ergänzend zum Weghalten der beatmeten Lunge vom Operationsgebiet. Alternativ oder zusätzlich kann der Retraktor optional auch einen weiteren Arbeitskanal enthalten, beispielsweise zur Einführung eines Lichtleiters zur Beleuchtung des OP-Feldes.

### Herstellung

Die Herstellung des erfindungsgemäßen Retraktors erfolgt in üblicher Weise aus biokompatiblen Materialien. Die Herstellung der massiven Kunststoffteile erfolgt bevorzugt im Spritzgussverfahren. Für die Metallteile wird bevorzugt rostfreier Edelstahl verwendet. Die Stoffbespannung des Retraktors erfolgt bevorzugt aus einem Nylon-Elastan-Gemisch (z.B.: 80% Polyamid (Nylon), 20% (Block-Copolymer aus Polyurethan und Polyethylenglycol (Elastan)). Es versteht sich von selbst, dass alle Teile des Retraktors sterilisierbar sein müssen.

Die in den Abbildungen beispielhaft dargestellte Ausführungsform eines erfindungsgemäßen Retraktors wird aus folgenden Teilen zusammengesetzt:
(Abbildung 1):
   - Instrumentenschaft (1)
   - Handgriff (2)
   - Spateleinheit (3)
   - Kaufteile (4)

Der Instrumentenschaft (1) besteht aus folgenden Bauteilen
(Abbildung 2):
   - Rechte Schafthälfte (1.1)
   - Linke Schafthälfte (1.2)

Der Handgriff (2) besteht aus folgenden Bauteilen:
(Abbildung 2):
   - Rechte Handgriffhälfte (2.1)
   - Linke Handgriffhälfte (2.2)
   - Endkappe (2.3)

Am Handgriff (2) befinden sich folgende Bedienelemente
(Abbildung 1):
   - Schieber (A)
   - Spannhebel (B)
   - Releaser (C)

Die Spateleinheit (3) besteht aus folgenden Bauteilen
(Abbildung 3):
   - Schubstange (3.1)
   - Innenrohr (3.2)
   - Zahnprofil (3.3)
   - Spreizblech (3.4)
   - Querriegel (3.5)
   - Spatelklammer (3.6)
   - Stoffhülle (3.7)

Das Instrument enthält die folgenden Kaufteile:
- PVC-Schlauch (ID x OD) 2 x 3,4 mm (4.1)
- Luerlock 71350 (4.2)
- Sicherungsring DIN 6799 - 1,9 (4.3)
- Edelstahlrohr 5,5 x 0,2 Wst. 1.4301 (4.4)

Alternative Ausführungsformen sind denkbar und ohne erfinderisches Zutun zu konstruieren.

## Patentansprüche

1. Chirurgischer Retraktor mit einem Schaft (1) und einem Griffteil (2), mit am distalen Ende des Griffteils (2) gelegenen aufspreizbaren Spatel (3) und mindestens einem weiteren Arbeitskanal zur Aufnahme einer Optik, wobei der aufspreizbare Spatel (3) am distalen Ende einer Schubstange (3.1) montiert ist, die sich in einem Rohr (3.2) befindet, wobei die Schubstange (3.1) sich relativ zum Griffteil (2) bewegen kann, wobei der Spatel (3) durch Spreizbleche (3.4) im Falle der Verkleinerung des Abstandes zwischen Rohr (3.2) und Schubstange (3.1) aufgespreizt wird,
wobei über den Spatel (3) ein hochelastischer Stoff gespannt ist, welcher im gespreizten Zustand eine geschlossene Oberfläche aufweist, durch die eine Kraftübertragung auf innere Organe ermöglicht wird.

2. Chirurgischer Retraktor gemäß Anspruch 1, wobei die Spreizbleche (3.4) im gespreizten Zustand arretiert werden.

3. Chirurgischer Retraktor gemäß Anspruch 2, wobei die Arretierung der Spreizbleche (3.4) durch Betätigung des Bedienelementes C kontrolliert aufgehoben wird, worauf die Spreizbleche (3.4) durch ihre Federwirkung spontan in den Ausgangszustand zurückversetzt werden, so dass der Retraktor geborgen werden kann.

4. Chirurgischer Retraktor gemäß Anspruch 1, wobei der über den Spatel gespannte hochelastische Stoff aus Polyamid, einem Block-Copolymer aus Polyurethan und Polyethylenglycol oder einer Mischung hieraus besteht.

5. Chirurgischer Retraktor gemäß Anspruch 1, **gekennzeichnet durch** mindestens einen weiteren Arbeitskanal.

6. Verwendung eines chirurgischen Retraktors gemäß mindestens einem der Ansprüche 1-5 in der Thorakoskopie.

7. Verwendung eines chirurgischen Retraktors gemäß mindestens einem der Ansprüche 1-5 im Rahmen der Implantation eines Herzschrittmachers.

8. Verwendung eines chirurgischen Retraktors gemäß mindestens einem der Ansprüche 1-5 in der Laparoskopie und Pelviskopie.
